# EUROPÄISCHE PATENTSCHRIFT

(11) **EP 0 931 540 B1**
(45) Veröffentlichungstag und Bekanntmachung des Hinweises auf die Patenterteilung: **29.08.2001**
(21) Anmeldenummer: 98124165.6
(22) Anmeldetag: 19.12.1998
(51) Int. Cl.: A61K 7/13, A61K 7/08, A61K 7/50

(54) **Verwendung von quaternären Ammoniumverbindungen in Mitteln zum Färben und Tönen von menschlichen Haaren**
Use of quaternary ammonium compounds in agents for dyeing and colouring human hair
Utilisation des composés d'ammonium quaternaire dans des agents de teinture et de coloration du cheveu humain

(30) Priorität: 31.12.1997 DE 19758271
(43) Veröffentlichungstag der Anmeldung: 28.07.1999
(73) Patentinhaber: GOLDWELL GmbH, 64297 Darmstadt (DE)
(72) Erfinder: Grit, Mustafa, Dr., 64579 Gernsheim (DE)

(56) Entgegenhaltungen:
- WO-A-97/16516
- WO-A-99/11227
- DE-A- 19 622 815

## Beschreibung

Die vorliegende Erfindung betrifft die Verwendung eines Mittels zum Färben bzw. Tönen von menschlichen Haaren, das eine Haarfärbung mit erhöhter Färbeintensität, Farbbrillanz und Farbbeständigkeit bewirkt.

Es ist allgemein bekannt, daß Haarfärbemittel in zwei Kategorien aufgeteilt werden, nämlich einerseits die permanenten Haarfärbemittel, die grundsätzlich Haarfarbstoffvorprodukte enthalten, die zusammen mit Oxidationsmitteln je nach Zusammensetzung die gewünschte Färbung auf dem Haar entwickeln; und andererseits semipermanente Haarfärbemittel, die direktziehende Farbstoffe enthalten, die zur Entwicklung ihrer Färbeleistung keinerlei Oxidationsmittelzusatzes bedürfen. Dementsprechend sind die Färbungen auch weniger dauerhaft als diejenigen mit Permanentfarbstoffen erzielbaren.

Diese Farbzusammensetzungen auf Basis direktziehender Farbstoffe werden in der Regel entweder als Tönungsshampoos, als Tönungslotionen oder als Tönungsfestiger, gegebenenfalls auch als Aerosolschaum, appliziert.

Die verwendeten direktziehenden Farbstoffe sind vorwiegend kationischer Natur; als weiteren wesentlichen Bestandteil enthalten die als Spülung verwendeten Zusammensetzungen auch kationische Tenside, insbesondere quaternäre Ammoniumsalze.

Tönungsshampoos enthalten anionische, nichtionische und/oder amphotere Tenside, vorzugsweise werden anionische im Gemisch mit nichtionischen und/oder amphoteren bzw. zwitterionischen Tensiden neben kationischen, direktziehenden Farbstoffen eingesetzt.

Die vorliegende Erfindung geht daher von der Aufgabenstellung aus, die Farbintensität und Farbstabilität von durch Anwendung direktziehender Haarfarbstoffe erzielten Färbungen zu verbessern.

Diese Aufgabe wird erfindungsgemäß dadurch gelöst, daß man Haarfarbe- und Haartönungsmitteln auf Basis direktziehender kationischer Haarfarbstoffe mindestens eine Verbindung der Formel I 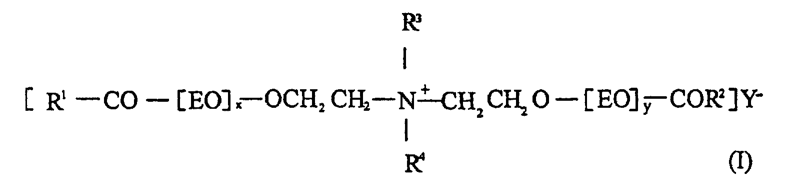 in der R¹ und R² jeweils für eine gegebenenfalls hydroxysubstituierte C₈-C₂₂- Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5, und Y⁻ für ein Anion stehen
zusetzt.

Bevorzugte Reste R¹ und R² sind C₁₂-C₁₈-Alkyl- und Oleylreste, der Rest R³ eine Methylgruppe und der Rest R⁴ eine Hydroxyethylgruppe. x, y und z sind vorzugsweise 0 oder 1; Y⁻ ist vorzugsweise ein Methosulfat-, Chlorid- oder Phosphatanion.

Die Menge an dieser Verbindung der Formel I liegt bei 0,1 bis etwa 20, insbesondere etwa 0,5 bis 15, vor allem etwa 1 bis etwa 10 Gew.-%, berechnet auf das Färbemittel.

Diese Verbindungen sind an sich bekannt und z.B. unter den Markenbezeichnungen "Schercoquat^{R}", "Tetranyl^{R}" und "Dehyquart^{R}F30" im Handel.

Aus der DE 196 22 815 A1 ist ein Haarbehandlungsmittel bekannt, das 0,1 bis 8 Gew.-% eines Difettsäuredialkanolaminoestersalzes enthalten und frei von kationischen Tensiden sein soll.
Dieses Mittel kann auch direktziehende Farbstoffe enthalten; es zeichnet sich durch seine Lagerstabilität, Verbesserung der Naßkämmbarkeit des Haares sowie gute Haut- und Augenverträglichkeit aus und verleiht dem Haar einen angenehmen Griff und einen schönen Glanz.
Die nicht vorveröffentlichte WO 99 11 227 A1 betrifft die Verwendung von Esterquats als Emulgatoren für Haarfarbstoffe, u.a. direktziehende Farbstoffe, zur Herstellung von Zubereitungen für die Färbung von Keratinfasern.

Der Anteil der direktziehenden Farbstoffe in den erfindungsgemäß verwendeten Zusammensetzungen liegt zwischen etwa 0,001 bis etwa 5, vorzugsweise 0,01 bis 2,5, insbesondere 0,1 bis 1 Gew.-% des Mittels.

Als direktziehende Haarfarbstoffe können im Prinzip alle für diesen Zweck vorgeschlagenen kationischen Farbstoffe verwendet werden.

Bevorzugt sind die sogenannten "Arianor"-Farbstoffe; vgl. K. Schrader, "Grundlagen und Rezepturen der Kosmetika", 2. Aufl. (1989), S. 811.

Besonders geeignete basische (kationische) Farbstoffe sind:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719. |

Selbstverständlich ist auch die Mitverwendung entsprechender direktziehender Pflanzenfarbstoffe oder auch anionischer direktziehender Haarfarbstoffe möglich.

Die erfindungsgemäßen Zusammensetzungen enthalten, insbesondere wenn es sich um Haarnachbehandlungsmittel wie z.B. Spülungen, Schäume, etc. handelt, vorzugsweise noch mindestens ein kationisches Tensid, insbesondere in einer Menge von 0,1 bis 7,5, vorzugsweise 0,25 bis 5, besonders bevorzugt 0,5 bis 2,5 Gew.-%, der Gesamtzusammensetzung.

Geeignete langkettige quaternäre Ammoniumverbindungen, die als kationische Tenside allein oder im Gemisch miteinander eingesetzt werden können, sind insbesondere Cetyltrimethylammoniumchlorid, Dimethyldicetylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Lauryltrimethylammoniumchlorid, Tris(oligooxyethyl)alkylammoniumphosphat, Cetylpyridiniumchlorid, Behentrimoniumchlorid etc.

Gut geeignet sind auch die in der EP-A 472 107 geoffenbarten quaternären Ammoniumsalze.

Im Prinzip sind alle quaternären Ammoniumverbindungen, wie sie im "CTFA International Cosmetic Ingredient Dictionary", Fourth Ed. (1991), unter dem Trivialnamen "Quaternium" aufgeführt sind, geeignet.

Die Zusammensetzung kann natürlich zusätzlich die in solchen Konditionierungsmitteln üblichen Bestandteile enthalten; es wird, zur Vermeidung von Wiederholungen, auf K.Schrader, "Grundlagen und Rezepturen der Kosmetika", 2. Aufl. (1989), S. 722 - 771, verwiesen.

Auch nichtionische Tenside können, insbesondere im Gemisch mit kationaktiven Tensiden, Verwendung finden, beispielsweise Aminoxide in einer Menge von etwa 0,25 bis etwa 5, vorzugsweise etwa 0,5 bis etwa 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder - ethylaminoxide, C₁₂-C₁₈-Alkyldi(hydroxyethyl)- oder -(hydroxypropyl)aminoxide, oder auch Aminoxide mit Ethylenoxid- und /oder Propylenoxidgruppen in der Alkylkette.

Geeignete Tenside sind weiterhin die bekannten C₈-C₁₈-Alkylpolyglucoside, insbesondere mit einem Polykondensationsgrad von 1,2 bis 3.

Wenn es sich bei den erfindungsgemäßen Zusammensetzungen um Shampoos handelt, können auch anionische Tenside, vorzugsweise im Gemisch mit nichtionischen, amphoteren und/oder zwitterionischen Tensiden, eingesetzt werden.

Geeignete anionische Tenside sind natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind α-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind auch Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 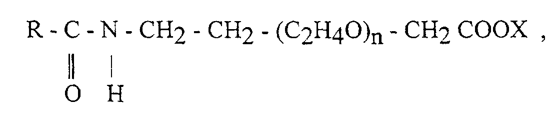 worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO" und "AKYPO-SOFT®".

Es ist besonders zweckmäßig, Mischungen aus mehreren anionischen Tensiden einzusetzen, beispielsweise ein Gemisch aus einem α-Olefinsulfonat und einem Sulfosuccinat, vorzugsweise im Verhältnis von 1 : 3 bis 3 : 1, oder einem Ethersulfat und einer Polyethercarbonsäure oder Alkylamidoethercarbonsäure.

Im Gemisch mit anderen anionischen Tensiden ebenfalls einsetzbar sind Eiweiß-Fettsäure-Kondensationsprodukte an sich bekannter Struktur, insbesondere in Mengen zwischen etwa 0,5 und 5, vorzugsweise 1 bis 3 Gew.-% der Gesamtzusammensetzung des flüssigen Haarwaschmittels.

Eine Übersicht über die in flüssigen Körperreinigungsmitteln zum Einsatz gelangenden anionaktiven Tenside findet sich im übrigen in der Monographie von K.Schrader, S. 683 bis 691.

Der bevorzugte Mengenbereich an anionischen Tensiden in den erfindungsgemäßen flüssigen Körperreinigungsmitteln liegt zwischen etwa 2,5 und etwa 25 Gew.-%, insbesondere bei etwa 5 bis etwa 20 Gew.-%, besonders bevorzugt bei etwa 7,5 bis etwa 15 Gew.-%, berechnet auf die Gesamtzusammensetzung des Mittels.

Nichtionische Tenside gelangen in Tönungsshampoos vorzugsweise im Gemisch mit anionaktiven Tensiden zum Einsatz.

Ein bevorzugtes nichtionisches Tensid gehört dabei, wie bereits oben erwähnt, zu der Klasse der Alkylpolyglucoside der allgemeinen Formel

R - O - (R¹O)ₙ - Zₓ ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 2,5 bedeuten.

Diese Alkylpolyglucoside sind in letzter Zeit insbesondere als ausgezeichnete hautverträgliche schaumverbessemde Mittel in flüssigen Wasch- und Körperreinigungsmitteln bekannt geworden und sind vorzugsweise in einer Menge von 1 bis 20, insbesondere 2,5 bis 15 Gew.-%, der Gesamtzusammensetzung enthalten.

Weitere nichtionische Tensidbestandteile in Tönungsshampoos sind beispielsweise langkettige Fettsäuremono- und -dialkanolamide, beispielsweise Cocosfettsäuremonoethanolamid und Myristinfettsäuremonoethanolamid, die auch als Schaumverstärker eingesetzt werden können.

Andere nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.

Gemische aus anionaktiven Tensiden und Alkylpolyglucosiden, den bevorzugten nichtionischen Tensiden im Rahmen der Erfindung sowie deren Verwendung in flüssigen Körperreinigungsmitteln sind an sich bereits bekannt, beispielsweise aus der EP-A 70 074. Die dort beschriebenen Alkylpolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere im Gemisch mit anionaktiven Tensiden einsetzbare Tenside sind die bereits erwähnten Aminoxide in einer Menge von etwa 0,25 bis etwa 5, vorzugsweise etwa 0,5 bis etwa 3,5 Gew.-%, berechnet auf die Gesamtzusammensetzung des Mittels.

Als amphotere bzw. zwitterionische Tenside können beispielsweise Betaine, Sulfobetaine und/oder Alkylaminocarbonsäuren wie Alkylaminoglycinate, Alkylaminopropionate, Alkylglycinate, etc. eingesetzt werden. Besonders geeignet sind Alkylamidobetaine der allgemeinen Formel 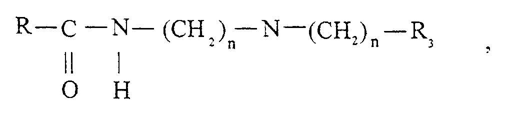 wobei R eine C₈-C₁₈-Alkylgruppe, z. B. einen Cocoalkylrest; R₁ und R₂ einen niederen C₁-C₄-Alkyl- oder -Hydroxyalkylrest, insbesondere eine Methyl-, Ethyl- und/oder Hydroxyethylgruppe; R₃ eine COO⁻-oder -SO₃⁻-Gruppe; und n 1 bis 3 bedeuten.

Auch Betaine der allgemeinen Formel 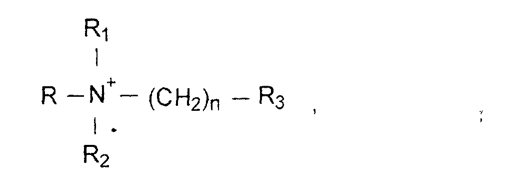 wobei R, R₁, R₂, R₃ und n die obengenannte Bedeutung haben, sind bevorzugt.

Geeignet sind insbesondere auch Handelsprodukte wie beispielsweise "Tegobetaine®", "Dehytone®" wie "AB 30", "G" und "K", "Lonzaine®", "Varion®" wie "ADG" und "CAS", "Lexaine®", "Chembetaine®", "Mirataine®", "Rewoteric®", "Schercotaine®", "Monteine LCQ®"; ""Alkateric®",
"Amonyl®", "Amphosol®", "Cycloteric BET®", "Emcol®", "Empigen®", "Mackam®", "Monateric®", "Unibetaine®" und "Velvetex®".

Die amphoteren bzw. zwitterionischen Tenside sind vorzugsweise in einer Menge von 0,25 bis 7,5 Gew.-%, insbesondere 0,5 bis 5 Gew.-%, berechnet auf die Gesamtzusammensetzung, enthalten.

Die erfindungsgemäßen Haarfärbemittel können die in solchen wäßrigen Zubereitungen üblichen Stoffe enthalten.

Dies sind beispielsweise synthetische oder natürliche haarkonditionierende Polymere, vorzugsweise in einer Menge von 0,1 bis 2,5, insbesondere 0,25 bis 1,5 Gew.-% der Gesamtzusammensetzung.

Als geeignete kationische Polymere sind neben den altbekannten quaternären Cellulosederivaten des Typs "Polymer JR" insbesondere quaternisierte Homo- und Copolymere des Dimethyldiallylammoniumchlorids, wie sie unter dem Handelsnamen "Merquat" im Handel sind, quaternäre Vinylpyrrolidon-Copolymere, insbesondere mit Dialkylaminoalkyl(meth)acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind, geeignet.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Anstelle der kationischen Polymeren oder in Kombination mit denselben können auch nichtionische Polymere verwendet werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat, wie sie beispielsweise von der Firma BASF unter dem Handelsnamen "Luviskol" vertrieben werden, eingesetzt.

Es können jedoch auch (Co-)Polymerisate aus den verschiedenen Acryl- und Methacrylestern, Acrylamid und Methacrylamid, beispielsweise Polyacrylamid mit Molgewichten von über 100.000, Dimethylhydantoin-Formaldehyd-Harze, etc., verwendet werden. Selbstverständlich sind auch Mischungen aus verschiedenen nichtionischen Polymeren verwendbar.

Geeignet sind schließlich auch noch amphotere Polymere, z. B. die unter der Bezeichnung "Amphomer" vertriebenen Copolymerisate aus N-Octylacrylamid, N-Butylaminoethylmethacrylat und Acrylsäure und die ebenfalls aus dem Stand der Technik hinreichend bekannten anionischen Polymeren.

Die erfindungsgemäßen Färbemittel können, wenn sie als Emulsionen, Dispersionen, Lösungen, Gele oder Aerosole, d.h., als Nachbehandlungsmittel eingesetzt werden, die in solchen Zusammensetzungen üblichen Zusätze enthalten, deren Art und Charakter von der Applikationsform des Mittels abhängig ist. Es sind dies Fette, Fettalkohole, UV-Absorber, insbesondere wasserlösliche anionische, Emulgatoren, pH-Regulatoren, Lösungs- und Verbindungsmittel, Lösungsvermittler, Konservierungsmittel, Parfums, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in den erfindungsgemäßen Zusammensetzungen vorzugsweise in einer Gesamtmenge von etwa 0,5 bis etwa 10, insbesondere etwa 1 bis 7,5, vor allem etwa 1,5 bis Gew.-%, berechnet auf die Gesamtzusammensetzung, enthalten.

Eine Zusammenfassung der Herstellung solcher Mittel findet sich in der bereits erwähnten Monographie von K. Schrader, S. 798 bis 815, insbesondere S. 804 ff.

Die erfindungsgemäßen Färbemittel liegen, soweit sie keine Tönungsshampoos sind, als Emulsion, Dispersion oder (gegebenenfalls verdickte, d. h. als Gel) Lösung vor und können auch als Aerosolschaum konfektioniert werden. Diese Zusammensetzungen und ihre Herstellung sind dem Fachmann grundsätzlich bekannt und bedürfen daher keiner näheren Erläuterung.

Der pH-Wert der erfindungsgemäßen Haarfärbemittel liegt vorzugsweise bei 3 bis 8, insbesondere zwischen 4 und 6.

Die folgenden Beispiele beschreiben die Zusammensetzung der erfindungsgemäßen Mittel.

### Beispiel 1

### Haartönungskonditioner

| | |
|---|---|
| Verbindung der Formel I (R¹=R²=Oleyl; x=y=0: R³=CH₃; R⁴=CH₂-CH₂-OH; Y⁻=CH₃S0₄⁻) | 2,00 (Gew.-%) |
| Cetylstearylalkohol | 5,00 |
| Isopropylmyristat | 0,50 |
| Hydroxypropylguarhydroxypropyltrimoniumchlorid | 0,40 |
| Panthenol | 0,50 |
| Isostearylglycerylpentaerythrylether | 0,20 |
| Citronensäure | 0,30 |
| Benzophenone-4 | 0,30 |
| Natriumhydroxid | 0,15 |
| Parfum | 0,40 |
| Konservierungsmittel | 0,15 |
| Basic Brown 17 | 0,12 |
| Basic Brown 16 | 0,06 |
| Basic Blue 99 | 0,05 |
| Wasser | ad 100,00 |

Beim Auftrag dieses Farbkonditioners auf menschliches Haar wurde eine glänzende Braunfärbung erzielt, die nach 3 Haarwäschen noch in gutem Zustand war. Der Ersatz der Verbindung nach Formel I durch die gleiche Menge Stearyltrimethylammoniumchlorid führte zu einer matten Braunfärbung, die bereits nach einer Haarwäsche deutlich verblaßt war.

### Beispiel 2

### Tönungsshampoo

| | |
|---|---|
| Natrium-C₁₂-C₁₄-alkylethersulfat (2-3 EO) | 10,00 (Gew.-%) |
| Cocoamidopropylbetain | 2,00 |
| PEG-3-distearat | 2,00 |
| Verbindung der Formel I (R¹=R²=Oleyl; R³=CH₃; R⁴=CH₂CH₂OH; x=y=0; Y⁻=CH₃SO₄⁻) | 1,00 |
| Basic Red 2 | 0,15 |
| Citronensäure, Konservierungsmittel | q.s. |
| Wasser | ad 100,00 |

Mit diesem Tönungsshampoo wurde eine ausdrucksvolle, intensive, dauerhafte glänzende Rotfärbung erzielt, die nach drei Haarwäschen noch in gutem Zustand war. Weglassen der Verbindung der Formel I führte zu einer wesentlich schwächeren Färbung, die bereits nach einer Haarwäsche verblaßt war.

### Beispiel 3

### Tönungsshampoo

| | |
|---|---|
| C₁₂-C₁₄-Alkylpolyglucosid (P.D.∼ 1,45 | 10,00 (Gew.-%) |
| Cocoamidopropylbetain | 2,00 |
| PEG-3-distearat | 2,00 |
| Verbindung der Formel I (R¹=R²=C₁₈H₃₇; R³=CH₃; R⁴=CH₂CH₂OH; x=y=0; Y⁻=Cl⁻) | 1,50 |
| Parfum | 0,40 |
| Citronensäure, Konservierungsmittel | q.s. |
| Basic Brown 16 | 0,10 |
| Basic Brown 17 | 0,10 |
| Basic Blue 99 | 0,02 |
| Wasser | ad 100,00 |

Es wurde eine ausdrucksvolle, glänzende, dauerhafte, intensive Braunfärbung erzielt, die auch nach drei Haarwäschen noch in gutem Zustand war.
Weglassen der Verbindung der Formel I führte zu einen Produkt, das eine matte Braunfärbung ergab, die nach nur einer Haarwäsche total verblaßt war.

## Patentansprüche

1. Verwendung von 0,1 bis 20 Gew.-%, berechnet auf die Gesamtzusammensetzung, mindestens einer Verbindung der allgemeinen Formel I 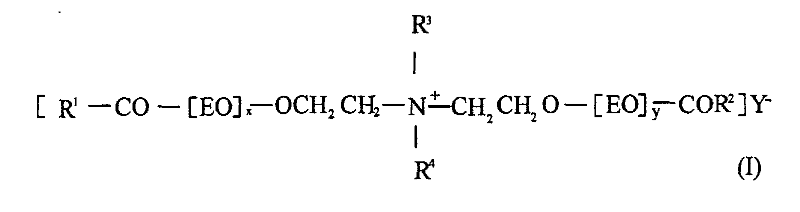 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}-H, sowie x, y und z für 0 bis 5, und Y⁻ für ein Anion stehen, zur Verbesserung der Farbstabilität und Farbintensität in Mitteln zum Färben und Tönen von menschlichen Haaren, die 0,001 bis 5 Gew.-% mindestens eines direktziehenden kationischen Haarfarbstoffs, berechnet auf die Gesamtzusammensetzung, enthalten.

2. Verwendung nach Anspruch 1, **dadurch gekennzeichnet**, daß die Reste R¹ und R² jeweils einen Oleylrest, der Rest R³ eine Methylgruppe, der Rest R⁴ eine Gruppe -CH₂-CH₂-OH, und x und y 0 bedeuten.

3. Verwendung nach Anspruch 1, **dadurch gekennzeichnet**, daß die Reste R¹ und R² jeweils eine C₁₂-C₁₈-Alkylgruppe, der Rest R³ eine Methylgruppe, und der Rest R⁴ eine Gruppe -CH₂-CH₂-O-[EO]_{z}H, und x, y und z 0 bedeuten.

## Claims

1. Use of 0.1 to 20% by wt., calculated to the total composition, of at least one composition of the general formula (I) 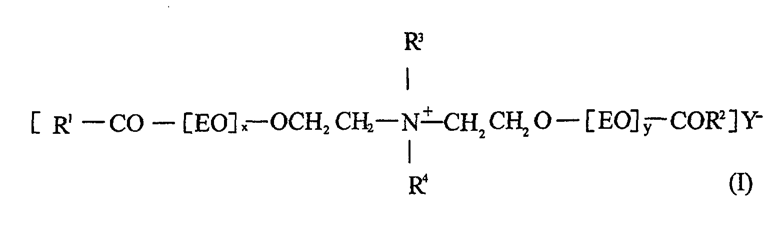 wherein R¹ and R² each are an optionally hydroxy-substitued C₈-C₂₂-alkyl- or alkenyl group, R³ and R⁴ are a C₁-C₃-alkyl group or a group -CH₂-CH₂-O-[EO]_{z}-H, x, y and z are 0 to 5, and Y is an anion, for improvement of color stability and intensity in compositions for dyeing and tinting of human hair, comprising 0.001 to 5% by wt., calculated to the total composition, of at least one direct-acting cationic hair dyestuff.

2. Use according to claim 1, **characterized in that** groups R¹ and R² each are an oleyl group, the group R³ is a methyl group, the group R⁴ is a group -CH₂-CH₂-OH, and x and y are 0.

3. Use according to claim 1, **characterized in that** the group R¹ and R² each are a C₁₂-C₁₈-alkyl group, the group R³ is a methyl group, and the group R⁴ is a group -CH₂-CH₂-O-[EO]_{z}H, and x, y and z are 0.

## Revendications

1. Utilisation de 0,1 à 20 % en poids, calculés par rapport à la composition totale, d'au moins un composé de formule générale I: 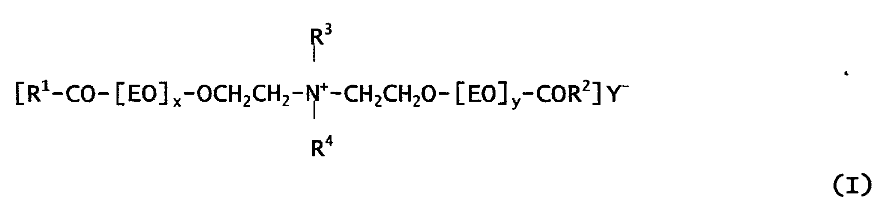 dans laquelle R¹ et R² représentent chacun un groupe alcényle ou alkyle en C₈ à C₂₂, éventuellement hydroxylé, R³ et R⁴ représentent un groupe alkyle en C₁ à C₃ ou un groupe -CH₂-CH₂-O-[EO]_{z}-H, x, y et z valant de 0 à 5 et Y⁻ représentant un anion,
pour l'amélioration de la stabilité et de l'intensité de la couleur de compositions de coloration et de teinture de cheveux humains qui contiennent de 0,001 à 5 % en poids, calculés par rapport à la composition totale, d'au moins un colorant pour cheveux direct cationique.

2. Utilisation selon la revendication 1, **caractérisée en ce que** les groupes R¹ et R² représentent chacun un groupe oléyle, le groupe R³ un groupe méthyle, le groupe R⁴ un groupe -CH₂-CH₂-OH, x et y valant 0.

3. Utilisation selon la revendication 1, **caractérisée en ce que** les groupes R¹ et R² représentent chacun un groupe alkyle en C₁₂ à C₁₈, le groupe R³ un groupe méthyle, le groupe R⁴ un groupe -CH₂-CH₂-[EO]_{z}H, x, y et z valant 0.
